(19) Europäisches Patentamt
European Patent Office
Office européen des brevets

(11) Publication number: **0 015 328**
**B1**

(12) **EUROPEAN PATENT SPECIFICATION**

(45) Date of publication of patent specification: **16.02.83**

(51) Int. Cl.³: **A 61 L 2/24**, A 61 L 2/06, A 61 L 2/00

(21) Application number: **79300192.6**

(22) Date of filing: **08.02.79**

(54) **Method and apparatus for steam sterilization.**

(43) Date of publication of application:
**17.09.80 Bulletin 80/19**

(45) Publication of the grant of the patent:
**16.02.83 Bulletin 83/7**

(84) Designated Contracting States:
**DE FR GB SE**

(56) References cited:
**FR - A - 1 401 080**
**US - A - 3 409 389**
**US - A - 3 436 170**
**US - A - 3 494 725**

(73) Proprietor: **American Sterilizer Company**
**2424 West 23rd Street**
**Erie, Pennsylvania 16512 (US)**

(72) Inventor: **Young, Jack, H.**
**R.D.3**
**Cambridge Springs Pennsylvania 16403 (US)**
Inventor: **Halleck, Frank E.**
**820 Elizabeth Lane**
**Erie Pennsylvania, 16505 (US)**

(74) Representative: **Jones, Colin et al,**
**W.P. THOMPSON & CO. Coopers Building Church Street**
**Liverpool L1 3AB (GB)**

**EP 0 015 328 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Method and apparatus for steam sterilization

This invention is concerned with steam sterilizing methods and apparatus.

Steam sterilization requires steam contact with every portion of the load to be sterilized (see Principles and Methods of Sterilization in Health Sciences", John J. Perkins, 2nd Edition, Third Printing, published by Charles C. Thomas, Springfield, Illinois, pages 110 to 114). Therefore proper conditioning of loads which include fabrics requires substantially complete removal of air from the interstices of the fabric in order to eliminate any air pockets and thus provide for heating the load throughout its volume.

Conditioning non-hermetically-sealed loads in prior steam sterilization practice has generally relied on high pre-vacuum treatment of steam pulsing. The high pre-vacuum treatment method relies on drawing a deep vacuum, to as low as a few millimetres of mercury in the load and the sterilizing chamber, in an attempt to substantially completely remove air from the load before the introduction of steam. This approach is costly, both in equipment and operation, since high-powered vacuum pumping apparatus is required and the entire system must have high vacuum integrity. Also, substantial time can be required to reach the high vacuum levels employed, especially with fabric-type loads.

Various steam pulsing methods have been taught (Principles and Methods of Sterilization in Health Sciences", *supra*, pages 150—152; U.S.A. Patent No. 3,409,389; and U.S.A. Patent No. 3,494,725), including steam pulsing above atmospheric pressure, steam pulsing below atmospheric pressure, and a combination of pulsing above and below atmospheric pressure. As set forth in "Principles and Methods of Sterilization in Health Sciences", *supra*, at page 151:

"The time required to heat the load to sterilizing temperature with a pulsing system depends upon the number of pulses and the time expended for each pulse. The effectiveness of a pulse, and therefore the number of pulses required, is in turn dependent upon the amplitude or excursion of the pulse and the ratio of the maximal and minimal absolute pressures of each pulse. The time expended by a pulse is a function of the rate of delivery and expulsion of steam and of the maximal and minimal pulsing pressures related to atmospheric pressure."

In the past, the conditioning portion of a steam cycle has been based on requirements to heat the most difficult load to be encountered in a particular sterilizer. In practice that sterilizer was then pre-set to carry out the conditioning cycle for the most difficult to heat load regardless of the actual characteristics or size of the load to be sterilized. In the steam pulsing method, a timer control or a pressure control functioned in a fixed manner, based on a fixed time or a fixed pressure excursion of the pulses. Such an approach can be inefficient when using fixed time cycles to sterilize partial loads or loads containing minimal fabric and when using fixed pressure cycles to sterilize full loads. Alternatives to a cycle based on the most difficult to heat load include operator prejudgement of loads and operator selection of the conditioning cycle, or use of conditioning sensors (e.g., temperature sensors) manually positioned within the load to monitor load conditioning. Such alternatives require operator steps and/or judgements and, therefore, are subject to oversight or error which good hospital practice attempts to avoid.

An object of the present invention is the provision of automated and accurate conditioning control which provides for monitoring the load without placement of load sensors in contact with the load and without requiring prejudgement of the load by the operator. The present invention resides in a method of steam sterilizing materials within a sealed sterilizing chamber in which a random load of such materials is loaded into the chamber and the load is conditioned prior to steam sterilization in that air is removed from the load and the load is heated to a desired temperature related to the sterilizing temperature by subjecting the chamber to a plurality of cyclic pressure pulses in each of which pressure is reduced during an evacuating phase and pressure is subsequently increased by introducing a vapour into the chamber during a vapour injecting phase, characterized in that the change of chamber pressure in relation to time lapse is measured during at least a portion of the evacuating phase, the measured change serving as an indication of chamber evacuation rate during the evacuating phase, and in that the indicated chamber evacuation rate automatically controls the termination of that evacuation phase and initiates the succeeding cyclic pressure pulse pressurization phase.

Thus, load characteristics are evacuated through rate of pressure change during cyclic pulsing to provide dependable and complete conditioning while enabling conservation of both time and energy. The method of the present invention is applicable to standard sizes of sterilizers used commercially and provide for complete conditioning to facilitate desired sterilization in the shortest practicle time and at minimum vacuum levels.

Partial or minimal fabric loads can present the greatest difficulty in obtaining proper air removal and complete conditioning automatically. For example, with a single fabric pack load, chamber pressure drops quickly so that, if evacuation is terminated at a fixed vacuum level, adequate exposure to vacuum may not be provided for proper removal of air which has been adsorbed on or is clinging to the fabric. Therefore, fixed pressure excursions during pulsing alone may not always provide the desired effectiveness or the efficiency of a reasonable number of pulses when working with all types of loads. With a full fabric load, chamber pressure drops more slowly so that the desired vacuum level may not be attained if evacuation is terminated on the basis of a fixed evacuation time.

Thus is carrying out the method of the present invention, data can be obtained to indicate characteristics of load being encountered to enable automated and accurate conditioning control, without requiring operator prejudgement of load or load contact sensors. The slope of the depressurization curve can be used during a cyclic pulse to evaluate the load. More specificially measurements of rate of depressurization may be co-ordinated to provide the functional input for a control logic decision on required parameters for proper conditioning of that load. In this way, vacuum exposure for proper air removal can be automatically and reliably provided for effective and efficient conditioning of both partial and full fabric loads.

Conditioning cyclic pulsing is provided through alternate evacuations and vapour pressurizations of the chamber. Since an objective is heating the load, the vapour should have high latent heat transfer upon condensation. Steam is the most commonly used vapour having transferable latent heat but other vapours, e.g., vapours of organic solvents, such as alcohols, ketones, and ethers, can be used in conjunction with steam. During the evacuation portion of a cyclic pulse, the slope of the draw-down curve can be utilized to provide an accurate evaluation of load characteristics to enable load responsive automatic control. The slope of the draw-down curve can be obtained by measurement of changes in chamber pressure versus time. No load contact monitors are required and data is obtained solely through chamber pressure and time measurements; that is, through instrumentation which is reliable under the harsh conditions of a sterilizer environment.

Air removal is important to proper load conditioning for steam heat sterilization. That is, air must be removed before steam can penetrate to condense and heat the load. The inter-relationship of various fabric-related properties to load characteristics is also significant. For example, percentage of fabric in the load, chamber volume utilization, and packing procedures all have a fabric-related effect. This effect is manifested as a composite which most closely, short of use of actual load contact sensors, indicates load characteristics for purposes of proper control of the conditioning cycle. This composite effect of fabric-related properties of a particular load is manifested most clearly while evacuating the chamber and is most accurately determined, and related to load conditioning, by measuring the rate of pressure change during selected portions of such evacuation.

In conditioning fabric-type loads prior to steam sterilization, there is a functional relationship between the slope of the evacuation phase pressure curve and the composite characteristics of the load. There is a functional relationship between the slope of the evacuation phase curve and the percent of fabric in a full combination fabric-and-hardgoods load or the fractional part of a load occupied by fabric whether in a full or partial load. Further, these two functional relationships closely correspond to one another so that, for control purposes, the composite characteristics of any load or partial load may be related to or considered as the percentage of a full load which is constituted of fabric.

The functional relationship between the slope of the evacuation phase pressure-time curve and the percentage of fabric can be established empirically for any sterilizer employing a given evacuation system. This relationship can then be employed to relate the slope of any such curve to a percentage of fabric which, in turn, can be employed to select the proper conditioning cycle and/or pressure-time parameters of the evacuation phase of the conditioning cycle for any load. For example, a curve representing this functional relationship may be established by plotting the percentage of fabric against the slope of the evacuation phase pressure-time curve for loads having various known percentages of fabric. Load-contact sensors and instrumentation, utilizing known test procedures, can be employed to monitor the conditioning of test loads to establish a curve representing the functional relationship and to determine the pressure and time requirements for conditioning.

The empirically established functional relationship can be provided as a program input to control logic for a sterilizer. The control logic can include a mini-computer or microprocessor which uses this input to automatically control load conditioning. To accomplish this, monitored time and pressure inputs to the control logic can be employed to compute the slope of the evacuation phase pressure-time curve which is compared with the functional relationship program input to determine the cycle and/or pressure-time parameters required for conditioning. This information can also be employed in other phases of an overall sterilizing cycle, as, for example, during drying where the fabric content of a load has a direct bearing on required drying time.

Minimum fabric loads can require longer or higher vacuum treatment for air removal during conditioning because the pressure drop in the chamber is very rapid for such loads. Conversely, the relatively slow pressure drop in a chamber containing a full fabric load provides longer exposure to vacuum so that lower vacuum levels provide adequate air removal. For example, for a full fabric load in a standard 914 mm sterilizer, an evacuation time of up to five minutes or more may be required for the chamber to reach a pressure of 0.67 Bar, absolute, whereas, with a minimum fabric load in the same sterilizer, the 0.67 Bar pressure may be reached in less than one minute.

While, in carrying out the method of the invention, conditioning may be controlled by controlling either time or pressure excursions, a combination of both may also be used. For example, time can be used as the controlling factor for minimum fabric loads, e.g., those loads which require less than one minute to reach a pressure of 0.67 Bar, absolute during evacuation, and pressure employed for controlling the evacuation phase of loads whose evacuation pressure-time curve slope indicates a higher percentage of fabric. This procedure avoids the necessity of using sophisticated and expensive vacuum measuring equipment capable of providing an analog output.

Pressure and time measurements can also be used directly in control without fixing the value of either. In a specific embodiment, minimum time and evacuation levels are established for each cyclic pulse. During the cyclic pulsing, only the upper pressure level, and consequently the upper temperature level, has a preselected or fixed value. The evacuation time and the lower pressure level can vary in a controlled manner.

Because of the data available in carrying out the method of the invention, the conditioning cycling can be optimized and the need for deep vacuum levels eliminated. Minimizing vacuum power requirements is an important contribution providing for use of effective, more economical and less complicated ejector apparatus in place of vacuum pumps. Reduction in both initial cost and operating power requirements result. This contribution of minimizing vacuum power requirements can be enhanced by flushing air from the chamber prior to cyclic pulsing by the early addition of steam in conjunction with evacuation. The efficiency of the ejector apparatus is increased due to removal of air and the collapse of steam and this also contributes to rapid and efficient heating of the load.

The invention includes sterilizing apparatus for steam sterilizing a random load of materials, the apparatus having a sealable sterilizing chamber capable of operation at pressure above atmospheric and being provided with evacuation means operable to evacuate the sterilizing chamber, vapour injection means operable to introduce a vapour into the sterilizing chamber, and a control device for controlling operation of the evacuation means and the vapour injection means during conditioning of the load prior to sterilization to provide a plurality of cyclic pulses of chamber pressure below an upper pressure level related to desired temperature for sterilization, each cyclic pulse including an evacuating phase during which pressure in the chamber is decreasing during operation of the evacuation means and a vapour injecting phase during which pressure within the chamber is increasing during operation of the vapour injecting means, characterized in that the control device is connected to a pressure sensing device for monitoring chamber pressure change and includes a timing device for measuring the time of the evacuation phase of the cyclic pulses, and in that the control device is responsive to such indication of chamber pressure and evacuation time for determining the rate of evacuation of the chamber and for controlling such cyclic pulsing to control load conditioning in response to the chamber evacuation rate.

In order to assure adequate conditioning during cyclic pressure pulsing, regardless of the nature of the load being conditioned, in accordance with one embodiment of the invention, both minimal evacuation time and minimal vacuum conditions must be met during the evacuation portion of a pulse before chamber evacuation is terminated and cyclic pressurization commenced. For example, sterilizers of the type and sizes generally employed in hospitals may be programmed to require an evacuation time of one minute and a pressure level of at most 0.67 Bar. Under these conditions, chamber evacuation continues for a period of one minute, regardless of the rate of evacuation or the vacuum level attained. At the end of the preselected one-minute time, chamber pressure is checked and, if the pressure is equal to or less than 0.67 Bar, evacuation is terminated and pressurization immediately commenced.

However, if the evacuation rate has been such that the pressure level is higher than 0.67 Bar at the conclusion of one minute, the evacuation continues until this desired pressure level is attained, regardless of the time requirement. Thus, for a heavy fabric load, the evacuation phase of a cyclic pulse may continue for four minutes or more before the desired subatmospheric pressure is attained; whereas, for a minimal fabric load the pressure within the chamber may be reduced to the minimal level prior to the expiration of one minute. By continuing evacuation for the required predetermined time for the minimal fabric loads, greater vacuum will be achieved, with the result that air within the load has an opportunity to escape and the pressure will be more nearly equalized throughout the load and chamber prior to steam pressurization.

The invention will be further described, by way of example, with reference to the accompanying drawings, in which:

Fig. 1 is a schematic representation of sterilizing apparatus for carrying out the invention;

Fig. 2 is a process flow chart for carrying out a sterilizing cycle utilizing the apparatus of Fig. 1;

Fig. 3 is a schematic representation of a control logic for controlling the sterilizing apparatus.

Fig. 4 is a graphic representation of pulse configuration achieved during load conditioning of a minimal fabric type load in the sterilizing apparatus; and

Fig. 5 is a graphic representation of the pulse configuration achieved during load conditioning of a full fabric type load in the sterilizing apparatus.

The steam sterilizing method of the invention can be practised with conventional sterilizer chamber configurations and sizes. As shown in Figure 1, a double-walled chamber structure 10 includes an inner, open-ended vessel 12 defining with an end closure, door 14, a sterilizing chamber 16. The vessel 12 is supported within an outer wall 18 which is spaced outwardly from and circumscribes a major portion of the vessel 12, an open end of the wall 18 is joined to the adjacent end of the inner vessel 12 by a flange 20 to define a steam jacket 22 between the inner and outer walls.

The door 14 can be hinged or otherwise mounted at one side of the open end of the double wall structure to facilitate opening and closing of the chamber 16. A conventional lock assembly can be used for the door 14, e.g., a camlock assembly, including an actuating lever or wheel 24 and locking cams 26, enabling the door to be firmly locked to tightly seal the chamber 16 during the sterilizing operation.

The jacket 22 and the chamber 16 are separately connected for discharge purposes, but interconnected for supply to provide certain advantages in load control. A drain pipe 28 connects the jacket 22 to a water-cooled heat exchanger 29 which discharges into a suitable drain. The heat exchanger 29 is supplied with cooling water which is mixed with the steam and hot condensate from the jacket to control the discharge temperature to a level acceptable for discharging into a conventional sewer drain. A steam trap 30 connected in the drain pipe 28 removes condensate from the jacket 22 and a one-way check valve 31 prevents possible back-flow through the pipe 28 into the jacket.

A second drain pipe 32 is connected between the vessel 12 and the heat exchanger 29. A steam trap 33 and a check valve 34 connected in the drain pipe 32 control the flow of steam condensate or other fluid from the chamber 16 through the heat exchanger 29 to waste. A temperature sensing bulb 36 is mounted upstream of the steam trap 33 and adjacent the outer chamber wall 18 to measure the temperature of steam flowing from the sterilizing chamber 16 for control of steam supply during sterilizing.

A main steam supply line or conduit 37 connects a source of steam, such as a boiler 38, to the steam jacket 22. A one-way check valve 39, a solenoid-actuated cut-off valve 40, and a pressure regulator 42 are connected in the main steam line 37 to control the flow of steam into the jacket 22. The steam line 37 can have a plurality of branches, not shown, for admitting steam into the jacket at a plurality of locations. The pressure regulator 42 co-operates with the steam trap 30 to maintain the pressure, and consequently the temperature, substantially constant in the jacket 22.

Steam is supplied to the chamber 16 from the steam jacket 22. To accomplish this, a pipe 44 is connected to the outer wall 18 with its inlet in communication with the steam jacket 22, and to the vessel 12 with its outlet in communication with the sterilizing chamber 16. A solenoid-actuated valve 45 is connected in the pipe 44 to control the flow of steam from the jacket 22 to the sterilizing chamber 16. Supplying steam to the chamber 16 from the jacket 22 avoids many of the problems of low quality steam frequently encountered in operating sterilizers. Condensate and the like is collected in the jacket and ultimately discharged from the bottom thereof through the drain pipe 28, so that only substantially saturated steam enters the chamber 16.

Chamber evacuation is accomplished through an evacuation conduit 46 connected to the drain line 32 upstream of the steam trap 33. The conduit 46 has its outlet connected to a water ejector 48. The ejector 48 is supplied with water, at a uniform pressure through a pipe 50 and a solenoid-actuated cut-off valve 52. Flow of water through the ejector 48 creates a suction in the end of the conduit 46 to thereby evacuate the sterilizing chamber 16. The water supply line 50 discharges into a suitable drain, with the steam evacuated from the chamber 16 being cooled by and condensed in the water before being discharged. Evacuator discharge can be supplied as the cooling fluid input to the heat exchanger 29 in order to conserve water. Flow through the evacuation conduit 46 is controlled by a solenoid-actuated valve 54 and a check valve 56.

An air supply line 58 is connected to the steam pipe 44 at a point downstream from the valve 45. Air at atmospheric pressure is supplied through the air line 58 and a filter 60 to the chamber 16 upon completion of a sterilizing cycle. The filter 60 is of a type to effectively remove bacteria as well as dust and the like from the air, and can also include air sterilizers. Flow through the filter and the air line is controlled by a solenoid-actuated shut-off valve 62 and a check valve 64.

A pressure sensor assembly 66, capable of sensing pressures both above and below atmospheric, is mounted within the chamber 16. The pressure sensor assembly 66 is connected, through suitable conductors, indicated generally at 68, to a master control logic 70 which controls operation of the apparatus.

Operation of the sterilization apparatus with cyclic pulses controlled to have a minimum evacuating time and reach a selected vacuum level is outlined in the flow chart of Figure 2. The cycle can include initially purging air from the chamber and then subjecting the material to a fixed number of cyclic pulses of pressure below a predetermined upper level by alternately evacuating the chamber and pressurizing it with conditioning vapour. At the conclusion of the conditioning pulsing, the chamber is pressurized with steam to a level producing the desired sterilization temperature, and maintained at this temperature (which can be accomplished by a pressure measurement if no air is present) for a predetermined time. Next, the sterilization chamber is evacuated and maintained at a relatively high vacuum for a predetermined time, for example five minutes, to dry the material. Finally, the chamber is brought to atmospheric pressure by the admission of air by passing atmospheric air through the bacterial filtering system.

It is preferred to initiate conditioning by introducing steam at super-atmospheric pressure to the sterilization chamber while simultaneously operating the evacuating system. When air is purged from the chamber in this way, pressure in the chamber initially increases until the increased proportion of steam in the steam-air mixture being evacuated increases the efficiency of the selected ejector system to the point that chamber pressure starts to decrease.

As part of the process depicted in Figure 2, this decrease in pressurization rate can be sensed and employed to generate a signal, with or without a time delay, closing the steam valve 45. Or a purge timer can be started at the initiation of steam injection or evacuation. Evacuation is continued, after the purge step, until the pressure sensor 66 senses that a predetermined subatmospheric pressure, for example 0.67 Bar absolute, has been reached and the time requirement has been satisfied. When these conditions have both been met, a conditioning pressure pulse counter is updated, the evacuating system is turned off, and the steam valve 45 is opened to admit steam into the chamber 16 from the jacket 22 to bring the pressure within the chamber up to the predetermined upper pressure level.

If the conditioning pulse counter signals that the load has been subjected to the proper number of pressure pulses, the conditioning phase of the cycle is ended. If the counter indicates that this number has not been reached, the steam valve 45 is closed, the evacuating system turned on, and the evacuating draw-down timer is again started to subject the system to another pressure pulse as just described. In the specific embodiment set forth, the load is subjected to a predetermined number of pressure pulses. Pulse counting can take place at lower or upper pressure levels.

In the later sterilization phase of the cycle, steam pressure within the chamber is maintained at the desired level, for example 2.8 to 3.1. Bar, absolute to maintain a required sterilization temperature for a predetermined time. At the end of the sterilization time, the evacuation system is turned on and operated for a predetermined time, preferably maintaining the vacuum during this time at the maximum level attainable by the evacuating system, to effectively dry the material in the chamber. Then the solenoid-actuated valve 62 in the air line 58 is opened, permitting filtered air to enter the chamber. When the pressure within the chamber is substantially equal to ambient atmospheric pressure, the cycle is complete and the door 14 can be opened.

The pressure sensor assembly 66 may be a conventional transducer capable of continuously sensing chamber pressure and transmitting an electrical signal proportional to the instantaneous pressure. This signal can then be used by the system control logic 70 to determine rate of pressure change, or the pressure-time curve slope, to control actuation of the various process phases. Alternatively, the pressure sensor assembly 66 can contain a plurality of sets of contacts, one which is actuated when the pressure within the chamber reaches a predetermined subatmospheric pressure, for example, 0.67 Bar, and a second which is actuated at the sterilization pressure, for example 2.8 Bar. These signals, in combination with the time signals from suitable conventional timers within the control logic 70, can be utilized to automate control of time and pressure parameters during the conditioning phase. Load responsiveness is thus provided by the vacuum levels and the time required to reach such levels which will depend on the nature of the load.

The electronic control logic illustrated in Figure 3, includes a conventional up-down counter 72 which may be up-counted by a signal on the "count" line when the "control" line is in a given state and down-counted by a signal on the "count" line when the "control" line is in a different given state. Before the process is begun, the up-down counter 72 is pre-loaded with a number which corresponds to the number of chamber evacuations to be employed.

Counter 72 can be loaded by depressing button 74 on pulse selector 76. Pulse selector 76 can be a conventional pulse generator arranged so that the number of times that button 74 is depressed appears at display window 78. Each time button 74 is depressed, pulse selector 76 is effective to generate a pulse on both the count line and the control line input to counter 72 and the counter is arranged so that it will up-count when a signal is present on the control line. In the embodiment shown, button 74 has been pressed to load the number three into counter 72. Up-down counter 72 can be automatically pre-loaded with the fixed number required, thereby removing from the operator's control any possible margin of error in improperly loading the up-down counter.

A start signal is generated by suitable means, such as by closing a conventional on-off switch connected in the main power circuit. When air is to be flushed from the chamber, the start signal causes the purge phase to begin. While the length of the purge and the maximum pressure attained during the purge phase may be controlled in a number of ways, as described above, Figures 3 to 5 illustrate the simplest mode of control, that is, a predetermined period of time.

The start signal is thus effective to start purge timer 80, which can be a conventional timer such as a digital timer, and which is arranged to emit a signal on the "finish" output line a predetermined period of time after a signal is fed to the "start" input line. The start signal input is also electrically connected to the electric control solenoid means of the valves 52 and 54 (Fig. 1) of the evacuation system and of the steam supply valve 45 to open these valves. Steam is admitted and the chamber is evacuated simultaneously. After a predetermined period of time, purge timer 80 times out and emits a signal on the "finish" output line which is effective to close the steam valve 45.

Referring to Figure 3, the "finish" output line of purge timer 80 is also connected to the "start" input of draw-down timer 82. The evacuation phase of the cyclic conditioning pulses continues until at least a predetermined vacuum level has been reached and a predetermined period of time has lapsed. In this embodiment, draw-down timer 82 can be started at the beginning of the steep negative-slope portion of the pressure curves of Figures 4 and 5 which corresponds to the time of closing of the steam valve 45. Other points can be used; starting the draw-down timer at about 1.3 Bar, absolute is representative.

Draw-down timer 82, for the minimum parameters embodiment being described, is similar to purge timer 80, and emits a signal on its output (or finish line) a predetermined period of time after a signal is fed to its input line. The output signal of draw-down timer 82 is fed to the set input of flip flop 84.

As stated above, the pressure sensing assembly 66 can include a first set of contacts arranged to emit signals when the upper preselected pressure is reached or exceeded and a second set which emits signals when the lower preselected pressure level is reached or exceeded. As shown in Figure 3, the signals are emitted on respective output lines, and the pressure sensing assembly 66 can comprise two separate pressure responsive switches or other sensing devices connected to respective signals generating circuits. When the lower preselected pressure is attained, a signal is emitted on line 86 which is fed to the set input of flip flop 88.

Set-reset flip flops 84 and 88 are effective to emit an output signal on their respective output lines when a signal is received on the set input lines, and to hold the signal on the output line until the flip flops are reset. The outputs of the flip flops are connected to the inputs of AND gate 90, and when both of the flip flops are set at the same time, AND gate 90 is effective to feed a signal to the "count" input of up-down counter 72. This will occur only when the pressure has reached or exceeded the predetermined low level and the draw-down timer 82 has timed out indicating that the negative-slope portion of the cycle has lasted for the predetermined minimum time. If only one of these two conditions is present, then there will be an output on only one of the flip flops, and consequently there will be no output signal at the output of AND gate 90.

The output of AND gate 90 is also connected to the steam valve 45 and to the evacuation valve 54, and is effective to respectively open the steam valve and close the evacuation valve, so that steam enters and pressurizes the chamber upon satisfaction of both the evacuation time and pressure parameters.

Assuming that the up-down counter 72 is not at zero, when the preselected higher pressure is exceeded, the pressure sensing assembly 66 is effective to generate an output signal on line 92 which also is connected to the evacuation valve 54 and the steam valve 45. This signal is effective to respectively open the evacuation valve and close the steam valve to cause the chamber pressure to drop. The occurrence of a signal on line 92 is further effective to set draw-down timer 82 which, as described above, emits an output signal on the "finish" line thereof when the predetermined time period has elapsed, and to reset flip flops 84 and 88.

The cyclic pulses continue in the above-described manner until the preselected number of cyclic pulses have occurred. The outputs of up-down counter 72 are connected to decoder 94, each output line of which is representative of a given number. Thus, at the time that the initial number of pulses is loaded into counter 72, the corresponding output line of decoder 94 has a signal thereon. When this initial number of pulses is counted down to zero, then the zero output line of decoder 92 has a signal thereon. This indicates the end of the conditioning phase, and the signal on the output of the zero line of the decoder, which is connected to a sterilizing timer 96, initiates the sterilizing phase.

Sterilizing timer 96 maintain an output signal on a line connected to the control solenoid of the steam shut-off valve 45 in the conduit 44 between the jacket 22 and the chamber 16. The timer 96 includes temperature level control means connected in the electrical line to the solenoid of the valve 45 to control the flow of steam into the chamber. The temperature level control means includes the temperature sensor 36, which transmits a signal proportional to the temperature of steam flowing from the chamber. The signal from the temperature sensor 36 is employed to control the opening and closing of the normally closed valve 45 to maintain the temperature within the chamber at the desired level within predetermined limits.

The pressure within the chamber 16 can also be employed to control opening and closing of the valve 45 during the sterilizing phase of the cycle. However, generally, temperature sensing means are preferred for economic reasons for controlling sterilizing steam flow.

When sterilizing timer 96 times out, a signal is emitted on its finish line to start a drying phase timer 98. Simultaneously, the steam valve 45 is closed and the vacuum valve 54 is opened. Drying timer 98 maintains the system in this condition for a predetermined time effective to dry the load. When drying timer 98 times out, it emits a signal on its finish line to close the evacuation system valves 52, 54. At the same time, the air valve 62 is opened, admitting filtered air into the chamber 16 to complete the cycle.

Figures 4 and 5 are graphic representations of chamber pressure, plotted against time, during operation of the apparatus described above in accordance with a preferred embodiment of the process of the invention wherein the purge phase of the sterilizing cycle is terminated after a predetermined time. Figure 4 represents the purging and conditioning phases of the cycle employed to sterilize a minimal fabric load, and Figure 5 represents operation of the same sterilizer with a full fabric load.

At the start of the cycles represented in both Figures 4 and 5, the introduction of steam under pressure into the chamber 16 while simultaneously operating the evacuating means results in a relatively sharp initial increase in pressure. With the minimal fabric load depicted in Figure 4, the pressure builds rapidly to above 2.4, Bar before the ejector apparatus is able to halt the pressure increase at point 100 on the pressure curve. At this point the pressure starts to fall gradually under the influence of the ejector while steam continues to be admitted. Purge timer 80, which as been set for a one-minute purge time, times out at point 102, and closes the steam valve 45. At the same time, draw-down timer 82 can be started.

The pressure drops from point 102 along curve section 104 to the minimum vacuum level (which has been pre-set at 0.67 Bar, absolute) at point 106. Approximately 1.3 minutes are required, after the purge phase, to reach the minimum vacuum level from point 102 in the embodiment shown. Thus, when draw-down timer 82 timed out at the end of one minute and emitted a signal to flip flop 84, flip flop 88 has not yet received a signal from the lower pressure contacts in the pressure sensing assembly 66. When this low pressure was sensed, resulting in a signal along line 86 to flip flop 88, AND gate 90 passed a signal to the up-down counter 72 to count it down one count, and simultaneously signalled the closing of the evacuation valve 54 and the opening of the steam valve 45. This condition remained stable along curve segment 108 from the minimum vacuum point 106 to the maximum chamber pressure of approximately 2.9 Bar at point 110. The pressure rise between points 106 and 110 is very rapid, following an almost straight line and requiring slightly less than one-half minute. The slope of the pressurization curve can be thought of as indicative of the heat absorption characteristics of the load while the evacuation phase slope is generally considered more indicative of air removal properties.

Upon sensing the maximum pressure, the pressure sensing assembly 66 emits a high pressure level signal along line 92 to simultaneously open the evacuating valve 54, close the steam valve 45, start draw-down timer 82 and re-set flip flops 84 and 88. In this condition, evacuation continued from point 110 along curve segment 112 to low pressure point 114. However, point 114 is at approximately 0.47 Bar, absolute, or approximately 0.2 Bar below point 106. This occurred because, when the pressure serving assembly 66 sensed the pre-set low pressure of 0.67 Bar and emitted a signal on line 86 to flip flop 88, the draw-down timer 82 had not yet timed out and emitted a signal to flip flop 84. Continued evacuation for a full minute resulted in the load within the chamber being subjected to an increased vacuum, thereby further assuring adequate withdrawal of air from the interstices of the fabric load.

This procedure was repeated for three full pressurization and evacuation pulses of the conditioning phase, at the conclusion of which the up-down counter was down-counted for the fourth time as the pressure within the chamber reached the point 116 on the curve. At this point, decoder 94 emitted a signal starting sterilizing timer 96 and opening the steam valve 45 to increase the pressure and the temperature within the sterilizer to the desired sterilizing temperature. The chamber pressure curve for the remaining portion of the cycle is not shown in Figures 4 or 5, since its configuration should be evident from earlier description.

Comparing curve section 104 of Figure 5 with the corresponding section 104 of Figure 4 demonstrates the automatic selection of conditioning tailored to the particular load. Thus, for the full fabric load of Figure 5, after one minute of evacuation time, from point 102, the pressure within the chamber 16 has only dropped to approximately atmospheric pressure. An additional 2.6 minutes, or a total of 3.6 minutes, evacuation time was required to reach a pressure of 0.67 Bar from 1.7 Bar, as compared with 1.3 minutes required to reach 0.67 Bar from approximately 2.5 Bar for the minimal fabric load. The minimal fabric load.

The relatively slower rise in chamber pressure during the flushing phase for the full fabric load, as compared with the minimal fabric load, is due to the differing absorptive characteristics of the loads.

Similarly, the substantially greater evacuation times required in Figure 5 result from the increased time required, especially during early pulses, to draw air from the interstices of the full fabric load. Subjecting the full fabric load to evacuation for an increased period of time results in a more efficient removal of air. However, such additional evacuation time is not required, and therefore not used, for the minimal fabric loads. The resulting savings in time and operating expenses should be apparent. It should also be apparent that a gradient of evacuating phase times is available between minimal fabric and full fabric loads and that the proper time for the particular load is automatically selected by this embodiment.

The load responsive aspects of the method make possible the use of relatively low level vacua while assuring proper air removal and heating throughout the load. As shown in Figure 5, a partial vacuum of only 0.67 Bar, absolute is satisfactory for carrying out the method of the invention, and the evacuation phase of each pressure pulse is terminated at this level. However, as shown in Figure 4, when utilizing this embodiment to condition a minimal fabric load, this minimum vacuum level may be reached before adequate air removal from any fabric present is achieved and, the load is subjected to a continuing vacuum, preferably at an increasing level, for a predetermined minimum time.

The load dependent aspects of the method are also evident from the pressurization phases which follow initial air flushing. Thus, from the initial low level point 106A of Figure 5 to the initial maximum or high pressure level 110A, the section of curve 108A requires slightly more than 2 1/2 minutes, as compared with less than one-half minute for the corresponding pressurization phase of the minimum fabric load in Figure 4. Also, the total time required to complete the conditioning phase, including the one-minute steam pressurization during flushing, and three complete pressurization-evacuation pulses is slightly less than seven (7) minutes for the minimum fabric load, as compared with almost twenty (20) minutes required for the full fabric load.

There are advantages, in addition to facilitating use of ejector apparatus instead of a vacuum pump, in the selection of a relatively low vacuum level objective (such as 0.67 Bar, absolute). Such low level vacuum increases the overall efficiency of a sterilizer and conserves energy by minimizing heat losses. High vacuum evacuation when the load is being heated by steam removes heat from the load and the sterilizer walls. Therefore, it is advantageous in decreasing heat losses to be able to condition the load with as little vacuum as possible consistent with rapid heat-up and proper air removal.

Pressure transducers can be used to monitor chamber pressure to provide information indicative of load characteristics and can be used, for the automatic selection of conditioning pressure and/or time, or even to select the cycle for conditioning that load. The functional relationship between the slope of the evacuation phase pressure-time curve for various loads processed in a particular sterilizer employing a given evacuation system can be programmed into the control logic and employed to select the desired conditioning pressure-time parameters based on a comparison of collected data with the functional relation derived empirically from preestablished or projected data from loads over a range of characteristics. The empirically established functional relationship is programmed into the control logic and employed as a basis for comparison of the slope of the evacuation phase curve computed from the monitored pressure input from the transducer and time input from conventional time signal generator means in the control logic.

Slope comparison for conditioning control preferably is carried out near atmospheric pressure in the evacuation phase, but average slope over a longer portion on all of the evacuation phase can also be employed. For example, it has been determined that, for a standard 914 mm sterilizer, when the slope of the evacuation pressure curve indicates an evacuation rate greater than about 0.62 Bar/min when determined between about 1.3 Bar and 1 Bar during an evacuation phase, the chamber pressure will reach a vacuum level of 0.67 Bar in less than one minute, and a control logic decision can then be made to continue the evacuation phase for a full minute. Conversely, a slope which indicates an evacuation rate less than 0.62 Bar/min, indicates that more than one minute will be required to reach a vacuum level of 0.67 Bar, and the control logic decision is to terminate the evacuation phase of the pulse only when this minimum vacuum level is reached.

Test loads for the evaluation of the method of the invention included sheet challenge packs, towel challenge packs, USA government surgical packs, and hardgoods packs.

Several criteria were evaluated in the qualification testing of conditioning and sterilizing by the method in accordance with the invention. Sterilization efficacy was demonstrated in full loads and single packs of fabrics, wrapped hardgoods, and unwrapped hardgoods. As an example of one test procedure, in each of five (5) consecutive cycles, biological indicators had to be rendered sterile, with an adequate safety margin demonstrated. The condition of the sterilized loads had to be dry, undamaged, and substantially free of visible wet-spotting on outer wrappers, or stains or residue on metal surfaces when recommended loading practices are followed. A further criterion was that all applicable government test specifications be achieved. For example, for a 610 mm×914 mm×1524 mm sterilizer, USA Federal specification GG—S—1343A specifies particular sized loads of surgical packs, and hardgoods, and also specifies allowable total cycle time for all such loads. Other criteria relating to sterility, drying criteria, pressure, and temperature tolerances were met.

These criteria were met while assuring that performance characteristics, production cost, and operating economies were all favourably comparable with present commercial sterilizers, when connected to utilities which would be encountered in normal operations.

During testing, temperatures were measured by a plurality of Type T (copper constant in) PTFE-insulated thermocouples, and pressure was monitored by Viatran absolute pressure measuring transducers. Temperature and pressure were continuously monitored and recorded.

Carefully tested biological and chemical indicators were packed in the test packs. The biological indicators were SPORDI biological spore strips produced by American Sterilizer Company of Erie, Pennsylvania. Chemical indicators used were American Sterilizer Company CHEMDI test indicators. These chemical indicators change colour to indicate complete load conditioning and steam penetration.

For evaluation test purposes thermocouples were packed in the central portion of the packs, in the vicinity of the chemical indicators and biological test strips, so that pack temperature were monitored along with chamber temperatures. In the case of hardgoods, the thermocouples were placed in direct metal-to-metal contact with the heaviest instruments or components of the pack, and chemical and biological indicators were distributed throughout the pack contents. Times were double-checked with stop-watches, with the times being compared to ensure repeatability.

In the test of five consecutive single pack and full loads of wrapped hardgoods, towel packs, sheet challenge packs, and ten-sheet USA government packs, sterility was produced in all test indicators processed. None of the packs processed exhibited any evidence of water spotting on the external wrappings following a cycle with a five-minute drying period. All fabric challenge packs were well within the allowable standards for drying efficiency.

Temperatures within the centre of the packs, for both full and minimal loads, were substantially equal to chamber temperatures before completion of the conditioning phase of the cycle.

Test cycles were run wherein the sterilization phase extended for a full recommended four minutes and for abbreviated periods. Biological test strips processed indicated sterility for both the full and abbreviated cycles, indicating an adequate margin of safety. Chemical indicators showed steam penetration throughout the loads in each case.

While specific embodiments of the invention have been described, the invention is not restricted to such embodiments.

## Claims

1. A method of steam sterilizing materials within a sealed sterilizing chamber in which a random load of such materials is loaded into the chamber and the load is conditioned prior to steam sterilization in that air is removed from the load and the load is heated to a desired temperature related to the sterilizing temperature by subjecting the chamber to a plurality of cyclic pressure pulses in each of which pressure is reduced during an evacuating phase and pressure is subsequently increased by introducing a vapour into the chamber during a vapour injecting phase, characterized in that the change of chamber pressure in relation to time lapse is measured during at least a portion of the evacuating phase, the measured change serving as an indication of chamber evacuation rate during the evacuating phase, and in that the indicated chamber evacuation rate automatically controls the termination of that evacuation phase and initiates the succeeding cyclic pressure pulse pressurization phase.

2. A method according to claim 1 characterised in that each of the evacuation phases is automatically terminated by automatically controlling the chamber evacuation pressure level.

3. A method according to claim 1 characterised in that each of the evacuation phases is automatically terminated by automatically controlling the chamber evacuation time.

4. A method according to claim 1, 2 or 3 characterised in that the range of pressure of the cyclic pressure pulses extends above and below atmospheric pressure.

5. A method according to claim 4 characterised in that the evacuation phase of each cyclic pressure pulse is continued until both a minimum vacuum level and minimum time duration are reached.

6. A method according to any preceding claim further characterised by selecting the number of cyclic pulses to control the conditioning of the load.

7. A method according to any preceding claim characterised in that the vapour introduced into the sterilizing chamber during the conditioning comprises steam.

8. A method according to any preceding claim characterised in that the load conditioning is initiated prior to the cyclic pressure pulses by purging air from the chamber by introducing the vapour into the chamber while simultaneously evacuating such vapour and air from the chamber.

9. A method as claimed in claims 7 and 8 characterised in that the air is purged from the chamber by introducing steam into the chamber at a rate to initially produce a substantial pressure increase in the chamber while simultaneously evacuating the chamber.

10. A method as claimed in claim 9 characterised in that steam is introduced into the chamber and the chamber is evacuated at rates to produce initially a rapid increase in chamber pressure followed by a substantial reduction in the rate of chamber pressurization and in that the change in the rate of chamber pressurization is sensed and the introduction of steam is terminated in response to the sensed reduction in rate of chamber pressurization.

11. A method as claimed in claim 9 characterised in that steam is introduced into the chamber and the chamber is evacuated at rates to produce initially a substantial increase in chamber pressure followed by a gradual reduction in chamber pressure, and in that the reduction in chamber pressure is sensed and the introduction of steam is terminated in response to the sensed pressure reduction.

12. A method according to any preceding claim characterised in that the pressure in the chamber is reduced during the evacuating phase by applying to the sterilizing chamber a vacuum by ejector means.

13. A method according to any preceding claim characterised in that the load conditioning comprises at least four cyclic pressure pulses.

14. Sterilizing apparatus for steam sterilizing a random load of materials, the apparatus having a sealable sterilizing chamber (16) capable of operation at pressure above atmospheric and being provided with evacuation means (48) operable to evacuate the sterilizing chamber, vapour injection means (37, 40) operable to introduce a vapour into the sterilizing chamber, and a control device (70) for controlling operation of the evacuation means and the vapour injection means during conditioning of the load prior to sterilization to provide a plurality of cyclic pulses of chamber pressure below an upper pressure level related to desired temperature for sterilization, each cyclic pulse including an evacuating phase during which pressure in the chamber is decreasing during operation of the evacuation means and a vapour injecting phase during which pressure within the chamber is increasing during operation of the vapour injecting means, characterised in that the control device (70) is connected to a pressure sensing device (66) for monitoring chamber pressure change and includes a timing device (82) for measuring the time of the evacuation phase of the cyclic pulses, and in that the control device (70) is responsive to such indication of chamber pressure and evacuation time for determining the rate of evacuation of the chamber and for controlling such cyclic pulsing to control load conditioning in response to the chamber evacuation rate.

15. Apparatus according to claim 14 characterised in that the control device (70) includes pulse cycle control means (84, 88, 90) for controlling such cyclic pulsing responsive to such indication of chamber pressure and evacuation time, whereby to continue each evacuation phase until both a predetermined time has lapsed and a predetermined vacuum level is reached in the sterilizing chamber.

16. Apparatus according to claim 14 or 15 characterised in that the control device (70) includes means (80) for initiating load conditioning by introducing steam into the chamber while simultaneously evacuating the chamber to flush air from the chamber.

17. Apparatus according to any of claims 14 to 16 characterised in that the vapour injector means (44) has a first electrically operated valve (45) to respectively pressurize the chamber (16) and stop the flow of steam into the chamber, and in that the evacuating means includes a suction device (48) and a second electrically operated valve (54) to respectively connect the chamber (16) to the suction device to evacuate the chamber and disconnect the chamber to stop the evacuation.

18. Apparatus according to any of claims 14 to 17 characterised in that the control device (70) includes counting means (72) operable to terminate the cyclic pressure pulsing after a predetermined number of such pulses.

**Patentansprüche**

1. Verfahren zur Dampfsterilisation von Materialien innerhalb einer abgedichteten Sterilisationskammer, bei dem eine beliebige Beladung dieser Stoffe in die Kammer eingegeben und die Beladung vor der Dampfsterilisation dadurch konditioniert wird, daß aus der Beladung Luft entfernt und die Belandung auf eine der Sterilisationstemperatur zugeordnete gewünschte Temperatur aufgeheizt wird, indem die Kammer mehreren zyklischen Druckimpulsen ausgesetzt wird, von denen in jedem der Druck während einer Evakuierungsphase verringert und anschließend durch Einführen eines Dampfes in die Kammer während einer Dampfinjektionsphase erhöht wird, dadurch gekennzeichnet, daß die Änderung des Kammerdrucks in bezug auf die verstreichende Zeit während mindestens eines Teils der Evakuierungsphase gemessen wird, wobei die gemessene Änderung als Anzeige der Evakuierungsrate der Kammer während der Evakuierungsphase dient, und daß die angezeigte Evakuierungsrate der Kammer automatisch die Beendigung der Evakuierungsphase steuert und die Druckbeaufschlagungsphase des nächstfolgenden zyklischen Druckimpulses einleitet.

2. Verfahren nach Anspruch 1, dadurch gekennzeichnet, daß jede der Evakuierungsphasen durch automatische Steuerung der Höhe des Evakuierungsdrucks der Kammer automatisch beendet wird.

3. Verfahren nach Anspruch 1, dadurch gekennzeichnet, daß jede der Evakuierungsphasen durch automatische Steuerung der Evakuierungszeit automatisch beendet wird.

4. Verfahren nach Anspruch 1, 2 oder 3, dadurch gekennzeichnet, daß der Druckbereich der zyklischen Druckimpulse sich über und unter den atmosphärischen Druck erstreckt.

5. Verfahren nach Anspruch 4, dadurch gekennzeichnet, daß die Evakuierungsphase eines jeden zyklischen Druckimpulses fortgesetzt wird, bis sowohl eine Mindestgröße des Vakuums als auch eine Mindestzeitdauer erreicht sind.

6. Verfahren nach einem der vorhergehenden Ansprüche, ferner dadurch gekennzeichnet, daß die Anzahl der zyklischen Impulse zur Steuerung der Konditionierung der Beladung gewählt wird.

7. Verfahren nach einem der vorhergehenden Ansprüche, dadurch gekennzeichnet, daß der in die Sterilationskammer während der Konditionierung eingeführte Dampf Wasserdampf enthält.

8. Verfahren nach einem der vorhergehenden Ansprüche, dadurch gekennzeichnet, daß die Konditionierung der Beladung vor den zyklischen Druckimpulsen durch Ausspülen der Luft aus der Kammer durchgeführt wird, indem der Dampf in die Kammer eingeleitet wird, während gleichzeitig dieser Dampf und Luft aus der Kammer evakuiert werden.

9. Verfahren nach den Ansprüchen 7 und 8, dadurch gekennzeichnet, daß die Luft aus der Kammer ausgespült wird, indem Luft in einer derartigen Menge in die Kammer eingeführt wird, daß zunächst ein wesentlicher Druckanstieg in der Kammer auftritt, während die Kammer gleichzeitig evakuiert wird.

10. Verfahren nach Anspruch 9, dadurch gekennzeichnet, daß Wasserdampf in die Kammer eingeführt wird und die Kammer mit derartigen Geschwindigkeiten evakuiert wird, daß zunächst ein schneller Anstieg des Kammerdrucks eintritt, an den sich eine wesentliche Verringerung des Maßes der Druckbeaufschlagung der Kammer anschließt, und daß die Änderung des Maßes der Druckbeaufschlagung der Kammer gemessen und die Einführung des Dampfes in Abhängigkeit von der gemessenen Verringerung des Maßes der Druckbeaufschlagung der Kammer beendet wird.

11. Verfahren nach Anspruch 9, dadurch gekennzeichnet, daß Dampf in die Kammer eingeführt und die Kammer mit solchen Geschwindigkeiten evakuiert wird, daß zunächst ein wesentlicher Anstieg des Kammerdruckes erzeugt wird, auf den eine stetige Verringerung des Kammerdrucks folgt, und daß die Verringerung des Kammerdrucks gemessen und das Einführen von Dampf in Abhängigkeit von der gemessenen Druckverringerung beendet wird.

12. Verfahren nach einem der vorhergehenden Ansprüche, dadurch gekennzeichnet, daß der Druck in der Kammer während der Evakuierungsphase durch Anlegen eines Vakuums durch eine Ejektorvorrichtung an die Kammer verringert wird.

13. Verfahren nach einem der vorhergehenden Ansprüche, dadurch gekennzeichnet, daß die Konditionierung der Beladung mindestens vier zyklische Druckimpulse umfaßt.

14. Sterilisationsvorrichtung für die Dampfsterilisation einer beliebigen Beladung aus Materialien, mit einer abdichtbaren Sterilisationskammer (16), die bei einem oberhalb Atmosphärendrucks liegenden Druck betrieben werden kann, und mit einer Evakuierungseinrichtung (48) zur Evakuierung der Sterilisierungskammer versehen ist, einer Dampfinjektionsvorrichtung (37, 40) zum Einführen eines Dampfs in die Sterilisationskammer und einer Steuereinrichtung (70) zur Steuerung der Operation der Evakuierungseinrichtung und der Dampfinjektionsvorrichtung während der Konditionierung der Beladung vor der Sterilisation zur Erzeugung mehrerer zyklischer Impulse des Kammerdrucks unter einer oberen Druckhöhe, die auf die für die Sterilisation gewünschte Temperatur bezogen ist, wobei jeder zyklische Impuls eine Evakuierungsphase, während der der Druck in der Kammer während des Betriebs der Evakuierungseinrichtung abfällt, und eine Dampfinjektionsphase, während der der Druck in der Kammer während des Betriebs der Dampfinjektionsvorrichtung ansteigt, aufweist, dadurch gekennzeichnet, daß die Steuereinrichtung (70) mit einer Druckfühlvorrichtung (66) zur Überwachung von Änderungen des Kammerdrucks verbunden ist und einen Zeitgeber (82) zur Bemessung der Zeit der Evakuierungsphase der zyklischen Impulse enthält und daß die Steuereinrichtung (70) auf diese Anzeige des Kammerdrucks und der Evakuierungszeit reagiert, um das Evakuierungsmaß der Kammer zu bestimmen und das zyklische Pulsieren zur Steuerung der Konditionierung der Beladung in Abhängigkeit von der Evakuierungsrate der Kammer zu steuern.

15. Vorrichtung nach Anspruch 14, dadurch gekennzeichnet, daß die Steuereinrichtung (70) eine Impulszyklus-Steuereinrichtung (84, 88, 90) zur Steuerung des zyklischen Pulsierens als Antwort auf die Anzeige des Kammerdrucks und der Evakuierungszeit enthält, wodurch jede Evakuierungsphase fortgesetzt wird, bis sowohl eine bestimmte Zeit verstrichen als auch eine bestimmte Größe des Vakuums in der Sterilisierungskammer erreicht ist.

16. Vorrichtung nach Anspruch 13 oder 14, dadurch gekennzeichnet, daß die Steuereinrichtung (70) eine Einrichtung (80) zur Einleitung der Konditionierung der Beladung durch Einleiten von Dampf in die Kammer bei gleichzeitiger Evakuierung der Kammer zum Austreiben von Luft aus der Kammer aufweist.

17. Vorrichtung nach einem der Ansprüche 14 bis 16, dadurch gekennzeichnet, daß die Dampfinjektionseinrichtung (44) ein elektrisch betriebenes erstes Ventil (45) für die Druckbeaufschlagung der Kammer (16) bzw. das Beendigen des Dampfstroms in die Kammer aufweist und daß die Evakuierungseinrichtung eine Saugvorrichtung (48) und ein elektrisch betriebenes zweites Ventil (54) aufweist, um die Kammer (16) zu ihrer Evakuierung mit der Saugvorrichtung zu verbinden bzw. zur Beendigung der Evakuierung die Verbindung zu unterbrechen.

18. Vorrichtung nach einem der Ansprüche 14 bis 17, dadurch gekennzeichnet, daß die Steuereinrichtung (70) eine Zähleinrichtung (72) enthält, die die zyklische Druckpulsierung nach einer bestimmten Zahl derartiger Impulse beendet.

**Revendications**

1. Procédé de stérilisation de matières à la vapeur d'eau dans une chambre de stérilisation formée, dans lequel une charge quelconque de matières est mise dans la chambre et, avant d'être stérilisée, est conditionnée, le conditionnement consistant à enlever l'air de la charge et à chauffer celle-ci à une température désirée en rapport avec la température de stérilisation en soumettant la chambre à une série d'impulsions cycliques de pression, lors de chacune desquelles la pression est abaissée pendant une phase de mise sous vide et ensuite augmentée par introduction d'une vapeur dans la chambre pendant une phase d'injection de vapeur, caractérisé par le fait que la variation en fonction du temps de la pression dans la chambre est mesurée pendant au moins une partie de la phase de mise sous vide, la variation mesurée servant d'indication de la vitesse de mise sous vide de la chambre pendant la phase de mise sous vide, et que la vitesse de mise sous vide indiquée commande automatiquement la fin de cette phase de mise sous vide et fait automatiquement commencer la phase de mise sous pression subséquente de l'impulsion cyclique de pression.

2. Procédé selon la revendication 1, caractérisé par le fait que chacune des phases de mise sous vide est terminée automatiquement par commande automatique de la pression de mise sous vide de la chambre.

3. Procédé selon la revendication 1, caractérisé par le fait que chacune des phases de mise sous vide est terminée automatiquement par commande automatique de la durée de mise sous vide de la chambre.

4. Procédé selon l'une des revendications 1 à 3, caractérisé par le fait que l'intervalle de pression des impulsions cycliques de pression s'étend au-dessus et au-dessous de la pression atmosphérique.

5. Procédé selon la revendication 4, caractérisé par le fait que la phase de production de vide de chaque impulsion cyclique de pression est poursuivie jusqu'à ce que soient atteints à la fois un niveau minimal de vide et une durée minimale.

6. Procédé selon l'une des revendications précédentes, caractérisé par le choix du nombre d'impulsions cycliques pour la commande du conditionnement de la charge.

7. Procédé selon l'une des revendications précédentes, caractérisé par le fait que la vapeur introduite dans la chambre de stérilisation pendant le conditionnement comprend de la vapeur d'eau.

8. Procédé selon l'une des revendications précédentes, caractérisé par le fait que le conditionnement de la charge comporte au début, avant les impulsions cycliques de pression, la purge d'air de la chambre par introduction de la vapeur dans la chambre et évacuation simultanée de cette vapeur et de l'air de la chambre.

9. Procédé selon les revendications 7 et 8, caractérisé par le fait que la chambre est purgée d'air par introduction de vapeur d'eau à un régime produisant initialement une élévation notable de la pression dans la chambre, et en même temps mise sous vide de la chambre.

10. Procédé selon la revendication 9, caractérisé par le fait que la vapeur d'eau est introduite dans la chambre et la chambre est mise sous vide à des régimes produisant initialement une élévation rapide de la pression dans la chambre suivie d'une réduction notable du régime de mise sous pression de la chambre et que le changement du régime de mise sous pression de la chambre est détecté et la fin de l'introduction de vapeur est produite d'après la réduction détectée du régime de mise sous pression de la chambre.

11. Procédé selon la revendication 9, caractérisé par le fait que la vapeur d'eau est introduite dans la chambre et la chambre est mise sous vide à des régimes produisant initialement une élévation notable de la pression dans la chambre suivie d'une baisse progressive de la pression dans la chambre, et que la baisse de la pression dans la chambre est détectée et la fin de l'introduction de vapeur est produite d'après la baisse détectée de la pression.

12. Procédé selon l'une des revendications précédentes, caractérisé par le fait que la pression dans la chambre est abaissée pendant la phase de production de vide par application à la chambre d'un vide au moyen d'un éjecteur.

13. Procédé selon l'une des revendications précédentes, caractérisé par le fait que le conditionnement de la charge comprend au moins quatre impulsions cycliques de pression.

14. Appareil de stérilisation pour la stérilisation à la vapeur d'eau d'une charge quelconque de matières, cet appareil comportant une chambre de stérilisation (16) pouvant être fermée hermétiquement et capable de fonctionner à des pressions supérieures à la pression atmosphérique, et étant pourvu d'un moyen (48) de production de vide dans ladite chambre, de moyens (37, 40) d'injection de vapeur pour l'introduction d'une vapeur dans ladite chambre, et d'un dispositif de commande (70) pour la commande du fonctionnement du moyen de production de vide et des moyens d'injection de vapeur pendant le conditionnement de la charge avant sa stérilisation, pour la production dans la chaùbre d'une série d'impulsions cycliques de pression au-dessous d'une pression supérieure, fonction de la température de stérilisation désirée, chaque impulsion cyclique comportant une phase de production de vide pendant laquelle la pression dans la chambre diminue pendant le fonctionnement du moyen de production de vide, et une phase d'injection de vapeur pendant laquelle la pression dans la chambre augmente pendant le fonctionnement des moyens d'injection de vapeur, caractérisé par le fait que le dispositif de commande (70) est relié à un dispositif capteur de pression (66) pour la détection de la variation de la pression dans la chambre et comporte un dispositif chronométrique (82) pour la mesure de la durée de la phase de production de vide des impulsions cycliques, et que le dispositif de commande (70) détermine le régime de production de vide dans la chambre d'après l'indication de la pression dans la chambre et de la durée de production de vide et commande les impulsions cycliques pour la conduite du conditionnement de la charge d'après le régime de production de vide dans la chambre.

15. Appareil selon la revendication 14, caractérisé par le fait que le dispositif de commande (70) comporte des moyens (84, 88, 90) de commande des impulsions cycliques d'après l'indication de la pression dans la chambre et de la durée de production de vide, en vue de la poursuite de chaque phase de production de vide jusqu'à ce qu'un temps déterminé soit écoulé et qu'un vide déterminé soit atteint dans la chambre de stérilisation.

16. Appareil selon l'une des revendications 14 et 15, caractérisé par le fait que le dispositif de commande (70) comporte un moyen (80) de mise en route du conditionnement de la charge par introduction de vapeur dans la chambre et mise sous vide simultanée de celle-ci pour en chasser l'air.

17. Appareil selon l'une des revendications 14 à 16, caractérisé par le fait que le moyen d'injection de vapeur (44) comporte une première électrovanne (45) pour la mise sous pression de la chambre (16) et l'arrêt de l'admission de vapeur dans la chambre, et que le moyen de production de vide comporte un dispositif d'aspiration (48) et une deuxième électrovanne (54) pour la connexion de la chambre (16) à ce dispositif d'aspiration pour sa mise sous vide et pour la déconnexion de la chaùbre pour l'arrêt de sa mise sous vide.

18. Appareil selon l'une des revendications 14 à 17, caractérisé par le fait que le dispositif de commande (70) comporte un moyen de comptage (72) ayant pour fonction de mettre fin aux impulsions cycliques de pression quand il en a été réalisé un nombre déterminé.

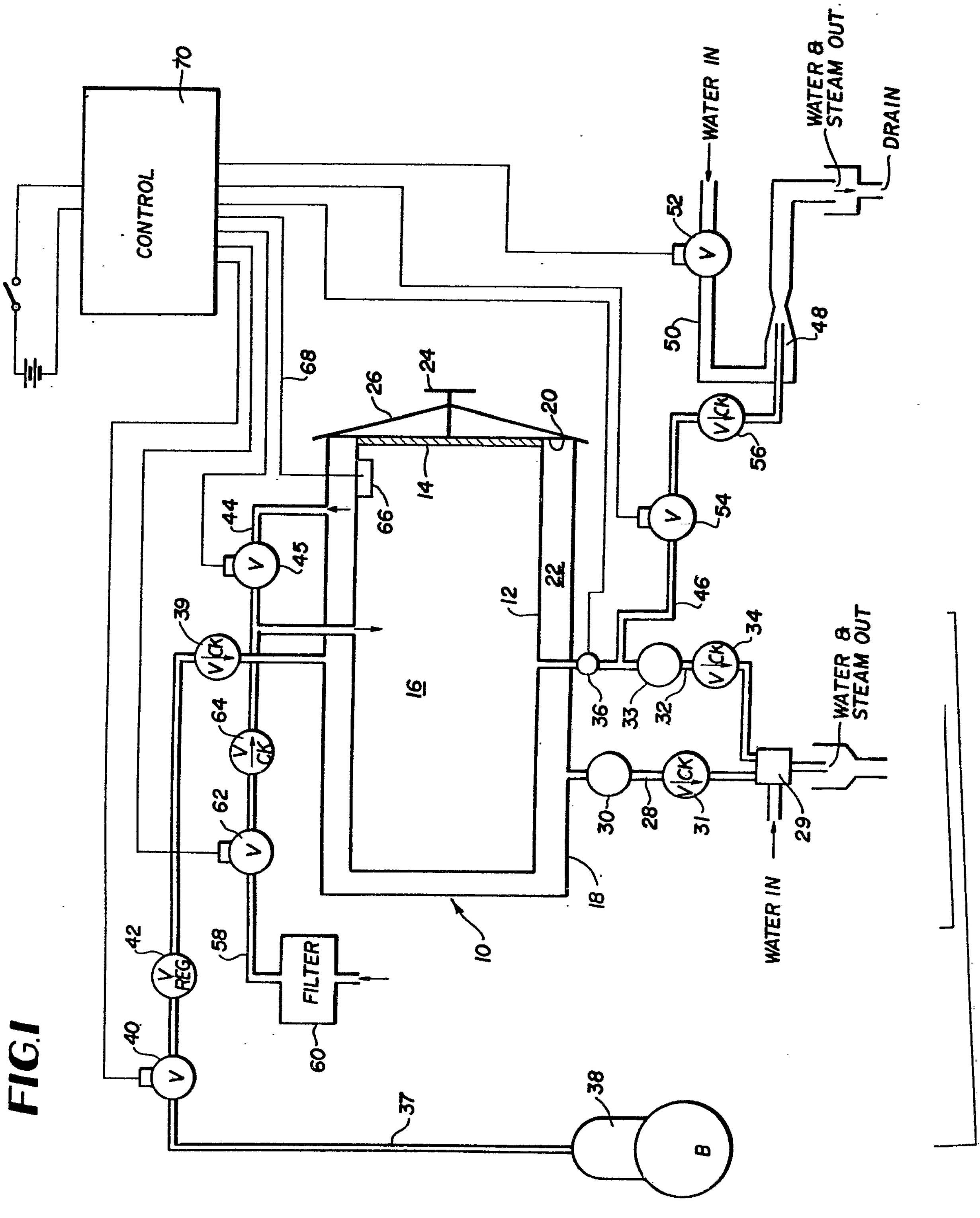
CONTROL
70
WATER IN
WATER & STEAM OUT
DRAIN
52
50
48
68
26
24
20
14
66
44
45
22
12
54
56
46
34
39
16
36
33
32
WATER & STEAM OUT
64
62
30
28
31
29
WATER IN
18
10
42
58
FILTER
60
40
37
38
B

FIG.I

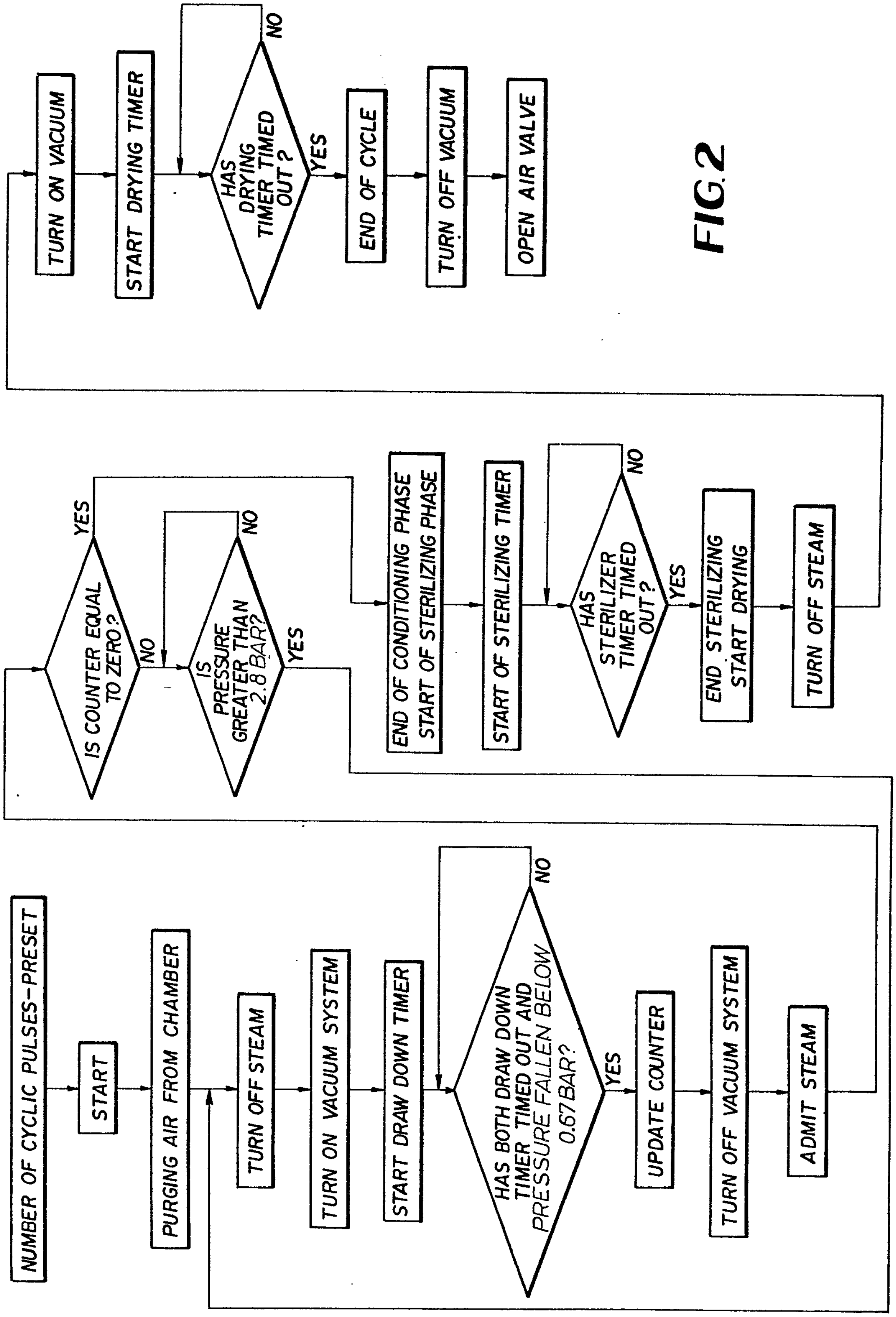
NUMBER OF CYCLIC PULSES-PRESET
START
PURGING AIR FROM CHAMBER
TURN OFF STEAM
TURN ON VACUUM SYSTEM
START DRAW DOWN TIMER
HAS BOTH DRAW DOWN TIMER TIMED OUT AND PRESSURE FALLEN BELOW 0.67 BAR?
NO
YES
UPDATE COUNTER
TURN OFF VACUUM SYSTEM
ADMIT STEAM
IS COUNTER EQUAL TO ZERO?
YES
NO
IS PRESSURE GREATER THAN 2.8 BAR?
NO
YES
END OF CONDITIONING PHASE START OF STERILIZING PHASE
START OF STERILIZING TIMER
HAS STERILIZER TIMER TIMED OUT?
NO
YES
END STERILIZING START DRYING
TURN OFF STEAM
TURN ON VACUUM
START DRYING TIMER
HAS DRYING TIMER TIMED OUT?
NO
YES
END OF CYCLE
TURN OFF VACUUM
OPEN AIR VALVE

FIG.2

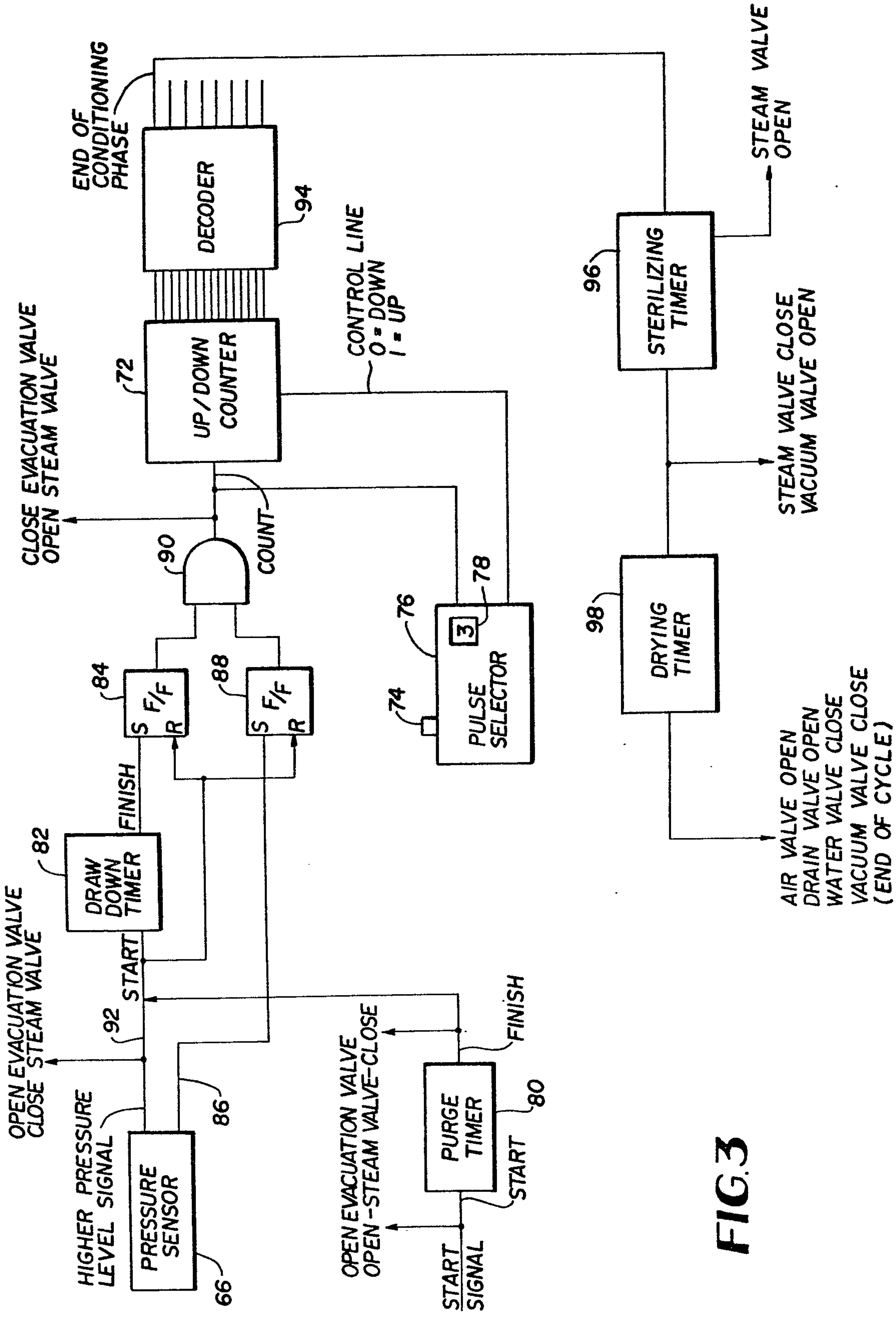
END OF CONDITIONING PHASE
DECODER
94
STEAM VALVE
STEAM VALVE OPEN
CONTROL LINE 0 = DOWN 1 = UP
CLOSE EVACUATION VALVE OPEN STEAM VALVE
72
UP/DOWN COUNTER
96
STERILIZING TIMER
STEAM VALVE CLOSE VACUUM VALVE OPEN
90
COUNT
76
78
3
84
F/F
S
R
88
F/F
S
R
74
PULSE SELECTOR
98
DRYING TIMER
AIR VALVE OPEN
DRAIN VALVE OPEN
WATER VALVE CLOSE
VACUUM VALVE CLOSE
(END OF CYCLE)
FINISH
82
DRAW DOWN TIMER
OPEN EVACUATION VALVE CLOSE STEAM VALVE
START
92
FINISH
OPEN EVACUATION VALVE OPEN-STEAM VALVE-CLOSE
HIGHER PRESSURE LEVEL SIGNAL
86
80
PURGE TIMER
START
PRESSURE SENSOR
66
START SIGNAL

FIG.3

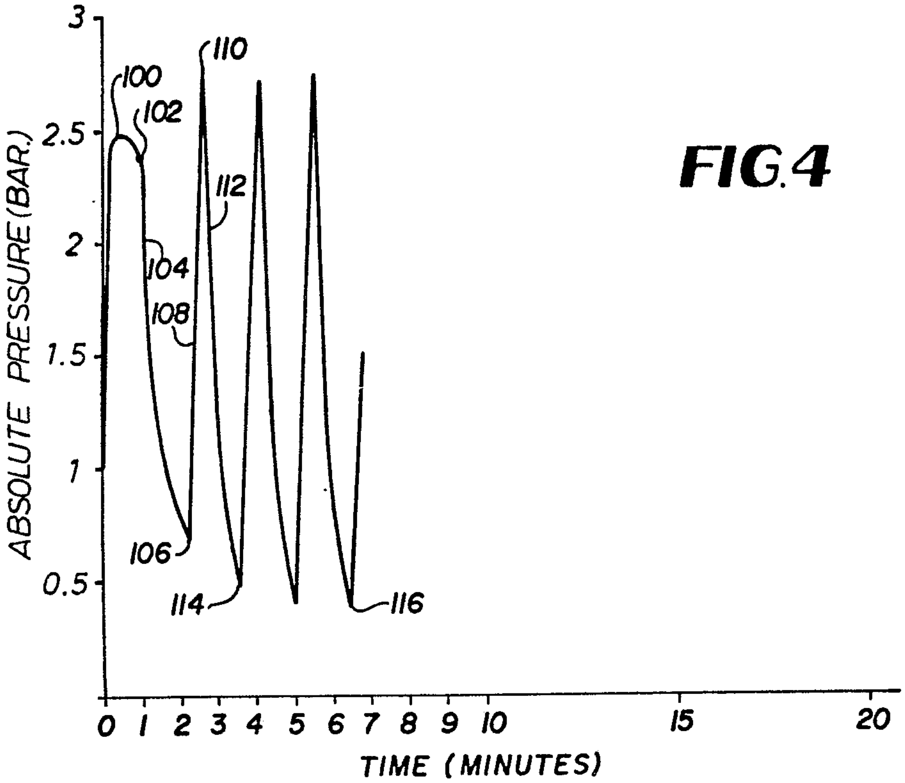
3
2.5
2
1.5
1
0.5
ABSOLUTE PRESSURE (BAR.)
100
102
104
106
108
110
112
114
116
0 1 2 3 4 5 6 7 8 9 10 15 20
TIME (MINUTES)

FIG.4

FIG.5

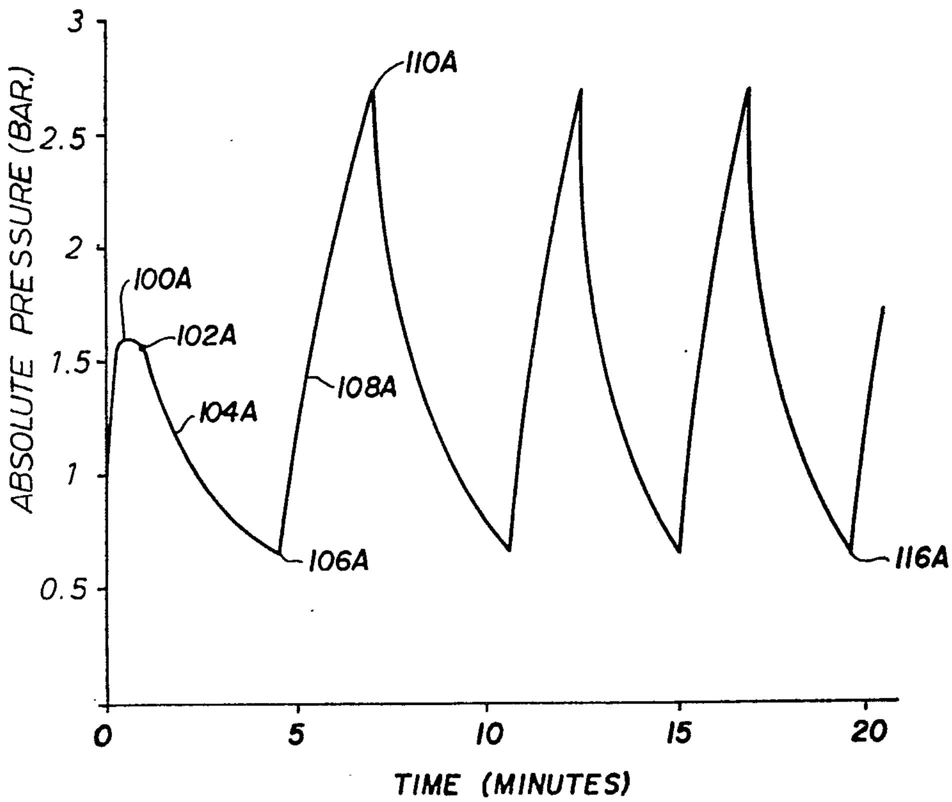
3
2.5
2
1.5
1
0.5
ABSOLUTE PRESSURE (BAR.)
100A
102A
104A
106A
108A
110A
116A
0 5 10 15 20
TIME (MINUTES)